# EUROPEAN PATENT APPLICATION

(11) **EP 2 623 055 A1**
(43) Date of publication of application: **07.08.2013**
(21) Application number: 11828807.5
(22) Date of filing: 15.09.2011
(51) Int. Cl.: A61B 17/56, A61F 2/28

(54) **PUNCTURE NEEDLE FOR INJECTING BONE CEMENT, AND METHOD FOR PRODUCING SAME**

(30) Priority: 28.03.2011 JP 2011069646; 28.09.2010 JP 2010217843
(71) Applicant: Terumo Kabushiki Kaisha, Tokyo 151-0072 (JP)
(72) Inventor: TAKIZAWA Kenji, Kawasaki-shi Kanagawa 216-8511 (JP); HAYAKAWA Koichi, Ashigarakami-gun Kanagawa 259-0151 (JP); UNO Takuya, Fujinomiya-shi Shizuoka 418-0015 (JP)
(74) Representative: TBK
(86) International application number: PCT/JP2011/071069
(87) International publication number: WO 2012/043247

(57) **Abstract**

Provided is a puncture needle (10) for injecting bone cement, comprising a side-port-forming member (18) embedded into an outer needle base (16). The outer needle base (16) is formed by insert molding. The side-port-forming member (18) covers the circumference of a second side hole (36), and provides communication between the second side hole (36) and the outside of the outer needle base (16). The method for producing the puncture needle (10) for injection of bone cement comprises affixing the side-port-forming member (18) to the basal end part of a double-tube member (58), and subsequently forming, by insert molding, an outer needle base for covering the side-port-forming member (18)

## Description

### Technical Field

The present invention relates to a puncture needle for injecting bone cement into the interior of a bone, as well as to a method for producing the same.

### Background Art

Percutaneous vertebral body plasty is a therapeutic method in which a vertebral body is reinforced by injecting bone cement into a damaged portion of the vertebral body for the purpose of removing pain arising from a compression fracture of the vertebral body. In recent years, percutaneous vertebral body plasty, which is a comparatively new therapeutic method that was carried out for the first time in France in 1987, has been practiced also in many institutions in Japan.

Percutaneous vertebral body plasty is basically a trans-pedicular approach in which a puncture needle of a hollow structure is made to puncture a vertebral body via pedicles located to the left and right on the back side of the vertebral body. Bone cement is injected into the vertebral body through an injection passage in the puncture needle. A bone biopsy needle is generally used as the puncture needle for injection of bone cement (see, for example, Japanese Laid-Open Patent Publication No. 2003-024339). The trans-pedicular approach includes a two-needle method, in which puncturing is conducted from both left and right sides, and a one-needle method, in which puncturing is conducted from only one side.

The one-needle method is advantageous over the two-needle method because it enables a reduction in cost, alleviation of complications, a reduction in the exposure dose, and shortening of the procedure time. On the other hand, there is a problem in that, when bone cement is injected by a conventional one-needle method, the internal pressure in the bone is raised accompanying injection of the bone cement, which may lead to leakage of bone cement to a location outside of the bone (for example, into the vertebral lumen or vein).

In order to solve such a problem, in International Publication No. WO 2010/044462, a puncture needle is proposed for injecting bone cement, in which an outer needle has a double-tube structure consisting of an inner tube and an outer tube. A first side hole is provided in the vicinity of a distal end portion of the outer tube, and a second side hole is provided in the vicinity of a proximal end portion of the outer tube. According to the puncture needle for injecting bone cement constituted in the foregoing manner, upon injection of bone cement into the bone through a hollow portion of the outer tube, gas or liquid (e.g., exudate or blood) is allowed to enter into a depressurization passage via the first side hole, whereupon the gas or liquid is discharged to the exterior of the living body via the second side hole. Consequently, it is possible to prevent the internal pressure inside the bone from rising due to injection of bone cement, as well as to prevent bone cement from leaking out to the exterior of the bone.

### Summary of Invention

Meanwhile, in the case that an outer needle base is provided on a proximal end portion (base portion) of the outer needle, which is provided with a side hole therein, similar to the puncture needle for injecting bone cement according to the aforementioned International Publication No. WO 2010/044462, it may be contemplated to form the outer needle base by insert molding. In this case, it is necessary to provide a countermeasure against generation of closure or deformation of the side hole during insert molding.

The present invention has been made taking into consideration the aforementioned circumstances. Accordingly, it is an object of the present invention to provide a puncture needle for injecting bone cement, in which an outer needle base can be formed by insert molding without inducing closure or deformation of a side hole, as well as to a method for producing the same.

In order to attain the above object, according to the present invention there is provided a puncture needle for injecting bone cement into the interior of a bone, including an outer needle of a hollow structure and having a bone cement passage therein, an outer needle base fixed to a proximal end portion of the outer needle and formed by insert molding, a side port-forming member embedded in the outer needle base, an inner needle having a needle tip at a distal end thereof, and which is inserted and passed in a hollow portion of the outer needle in a slidable manner, and an inner needle base secured to a proximal end portion of the inner needle. The outer needle includes a first side hole located on an outer circumferential portion in the vicinity of a distal end portion thereof, a second side hole located on an outer circumferential portion in the vicinity of the proximal end portion thereof, and a depressurization passage, which is independent from the bone cement passage, and which provides communication between the first side hole and the second side hole. The side port-forming member covers a circumference of the second side hole and provides communication between the second side hole and a location outside of the outer needle base.

According to the aforementioned constitution, the second side hole, which is provided on the proximal end portion of the outer needle, is covered by the side port-forming member, and the outer needle base is formed so as to cover the side port-forming member. Therefore, during molding of the outer needle base, the outer needle base can be insert-molded without causing closure or deformation of the second side hole.

In the aforementioned puncture needle for injecting bone cement, preferably, the side port-forming member includes a connecting section in which the outer needle is inserted, and a hollow port body section extending outwardly from a side portion of the connecting section, the second side hole confronts a proximal-end-side opening of the port body section, and the proximal-end-side opening of the port body section is greater than the second side hole.

According to the aforementioned constitution, the proximal-end-side opening of the port body section is greater than the second side hole. This ensures that during the production process, the second side hole can be placed so as to confront the proximal-end-side opening, without requiring accurate positional adjustment of the proximal-end-side opening and the second side hole. Thus, the position adjusting operation can be simplified.

In the aforementioned puncture needle for injecting bone cement, preferably, the side port-forming member includes a connecting section in which the outer needle is inserted, and a hollow port body section extending outwardly from a side portion of the connecting section, the connecting section is provided on an inner circumferential portion with a groove extending in a circumferential direction, and the second side hole confronts the port body section or the groove.

According to the aforementioned constitution, at a time of inserting the proximal end portion of the outer needle into the side port-forming member during a production process, there is no need for positional adjustment in the circumferential direction of the proximal-end-side opening of the port body section and the second side hole. Accordingly, the insertion operation can be simplified.

In the aforementioned puncture needle for injecting bone cement, preferably, a bulge-forming member, which bulges from an outer circumferential surface of the outer needle in order to make a cross sectional profile orthogonal to an axis of the outer needle non-circular, is secured to the outer needle, and the bulge-forming member is embedded in the outer needle base.

According to the aforementioned constitution, the bulge-forming member secured to the cross sectional profile of the outer needle is non-circular. Therefore, relative rotation of the outer needle and the outer needle base can be prevented from occurring, even if a high torque is exerted between the outer needle and the outer needle base. In addition, the bulge-forming member can prevent the outer needle from slipping out in a distal end direction from the outer needle base.

In the aforementioned puncture needle for injecting bone cement, preferably, the bulge-forming member is formed from a member that is C-shaped in cross section and extends along an axial direction of the outer needle. End portions of the C-shaped form of the bulge-forming member and an outer circumferential portion of the outer needle are joined together along the axial direction of the outer needle.

The bulge-forming member constituted in the foregoing manner is C-shaped in cross section, which makes it possible to easily make the cross sectional profile thereof non-circular. In addition, since joint parts are provided along the end portions of the C-shaped form, the bulge-forming member can be firmly secured to the outer needle.

In the aforementioned puncture needle for injecting bone cement, preferably, the bulge-forming member is formed from a tubular member the cross sectional profile of which is non-circular.

According to the aforementioned constitution, the bulge-forming member can be constituted using a simple tubular member. In addition, it is possible to easily provide a structure that bulges from an outer circumferential surface, and which makes the cross sectional profile orthogonal to the axis of the outer needle non-circular.

In the aforementioned puncture needle for injecting bone cement, preferably, the bulge-forming member is disposed more toward a distal end side of the outer needle than the side port-forming member.

According to the aforementioned constitution, the bulge-forming member is provided at a position on the outer needle base that is near the distal end side of the outer needle. At such a position, separation of the outer needle base and the outer needle is inhibited. Therefore, separation can be effectively prevented from spreading to the proximal end side of the outer needle base. Accordingly, relative rotation of the outer needle and the outer needle base can reliably be prevented.

In the aforementioned puncture needle for injecting bone cement, preferably, a flange part formed by plastic working is provided on a proximal end portion of an outer tube constituting the outer needle, and a projection, which is formed by plastic working and makes a cross sectional profile orthogonal to an axis of the outer tube non-circular, is provided on an outer circumferential portion of a part of the outer tube, the part being embedded in the outer needle base and located on a distal end side relative to the flange part.

According to the aforementioned constitution, using the flange part, it is possible to prevent the outer needle from slipping out in the distal end direction of the outer needle base. In addition, the projection that makes the cross-sectional profile non-circular ensures that relative rotation of the outer needle and the outer needle base can be prevented from occurring, even if a high torque is exerted between the outer needle and the outer needle base. Further, since the flange part and the projection are formed by subjecting the outer tube to plastic working, the structure for preventing slipping-out and rotation can easily be provided without the need for any additional component parts.

In the aforementioned puncture needle for injecting bone cement, preferably, the projection is provided more toward a distal end side of the outer needle than the side port-forming member.

According to the aforementioned constitution, the projection is provided at a position in the outer needle base, which is near the distal end side of the outer needle. Also, at such a position, mutual separation of the outer needle base and the outer needle from each other is inhibited. Therefore, separation can be prevented from spreading to the proximal end side of the outer needle base. Accordingly, relative rotation of the outer needle and the outer needle base can be prevented more securely.

In the aforementioned puncture needle for injecting bone cement, preferably, the side port-forming member is composed of a transparent member.

According to the aforementioned constitution, when liquid that has flowed out of a bone flows into the side port-forming member, the liquid inside the side port-forming member can visually be confirmed from outside the side port-forming member. Thus, flowing-out of liquid from the bone can be visually checked at an early time, which contributes to smooth progress of the injection technique.

In addition, according to the present invention, there is provided a method for producing a puncture needle for injecting bone cement into the interior of a bone, wherein the method includes a securing step in which a hollow side port-forming member having a side port at a distal end thereof is secured to a proximal end portion of a tubular member, so as to cover a circumference of a second side hole, the tubular member including a hollow portion that penetrates in an axial direction thereof, a first side hole provided on an outer circumferential portion in the vicinity of a distal end portion thereof, the second side hole provided on an outer circumferential portion in the vicinity of the proximal end portion thereof, and a depressurization passage, which is independent from the hollow portion, and which provides communication between the first side hole and the second side hole. The method also includes an outer needle base-forming step for forming, by insert molding, an outer needle base that covers the side port-forming member as well as the proximal end portion of the tubular member.

Herein, the above-mentioned tubular member is a concept, which includes not only an outer needle formed into a finished shape, but also an intermediate product prior to forming the outer needle into the finished shape. In the embodiments, the term "double-tube member" corresponds to such an intermediate component part.

According to the aforementioned production method, the outer needle base is insert-molded after the second side hole has been covered by the side port-forming member. Therefore, the outer needle base can be insert-molded without causing closure or deformation of the second side hole.

In the method of producing a puncture needle for injecting bone cement, preferably, in the securing step, the side port-forming member and the tubular member are adhered to each other by a heat-resistant adhesive.

By using a heat-resistant adhesive in this manner, securing of the side port-forming member and the outer tube to each other can be maintained assuredly, since the adhesive properties of the adhesive are not lost even if the adhesive is exposed to heat during insert molding of the hub body.

In the aforementioned method for producing a puncture needle for injecting bone cement, preferably, the side port-forming member is composed of a transparent member, and the securing step further includes a step of applying a UV (ultraviolet)-curable adhesive between the side port-forming member and the tubular member, and a step of irradiating the applied UV-curable adhesive with UV rays in order to cure the UV-curable adhesive.

By using a UV-curable adhesive in this manner, it is possible for the side port-forming member and the tubular member to be secured to each other in a short time.

In the aforementioned method for producing a puncture needle for injecting bone cement, preferably, the method further includes a bulge-adding step in which a bulge-forming member for making a cross sectional profile orthogonal to an axis of the tubular member non-circular is secured to an outer tube constituting the tubular member. Further, in the outer needle base forming step, the outer needle base is formed so as to cover the bulge-forming member.

According to the aforementioned constitution, the cross-sectional profile is made non-circular by adding the bulge-forming member to the outer tube. This ensures that relative rotation of the outer needle and the outer needle base can be prevented from occurring, even if a high torque is exerted between the outer needle and the outer needle base. In addition, due to the bulge-forming member, it is possible to prevent the outer needle from slipping out in the distal end direction of the outer needle base.

In the aforementioned method for producing a puncture needle for injecting bone cement, preferably, the method further includes a flange part-forming step in which a flange part is formed by plastic working on a proximal end portion of an outer tube constituting the tubular member, and a projection-forming step in which a projection, which makes a cross sectional profile orthogonal to an axis of the outer tube non-circular, is formed by plastic working on an outer circumferential portion on a distal end side relative to the proximal end portion of the outer tube. Further, in the outer needle base-forming step, the outer needle base is formed so as to cover the flange part and the projection.

According to the aforementioned method, the flange part makes it possible to prevent the outer needle from slipping out in the distal end direction of the outer needle base. In addition, the projection, which makes the cross-sectional profile non-circular, ensures that relative rotation of the outer needle and the outer needle base can be prevented from occurring, even if a high torque is exerted between the outer needle and the outer needle base. Further, since the flange part and the projection are formed by subjecting the outer tube to plastic working, the structure for preventing slippage and rotation can easily be provided without the need for any additional component parts.

According to the puncture needle of the present invention for injecting bone cement, together with the method for producing the same, the second side hole, which is provided on the proximal end portion of the outer needle, is covered by the side port-forming member, and the outer needle base is formed so as to cover the side port-forming member. Therefore, during a procedure of molding the outer needle base, the outer needle base can be insert-molded without causing closure or deformation of the second side hole.

### Brief Description of Drawings

FIG. 1 is a general perspective view of a puncture needle for injecting bone cement according to a first embodiment of the present invention;
FIG. 2 is a partially omitted sectional view of the puncture needle for injecting bone cement shown in FIG. 1;
FIG. 3 is a partially omitted sectional view of the puncture needle for injecting bone cement shown in FIG. 1, in a condition in which an inner needle has been pulled out of an outer needle;
FIG. 4A is a partially omitted sectional view showing a double-tube member constituting the outer needle of the puncture needle for injecting bone cement;
FIG. 4B is a partially omitted sectional view of a condition in which a side port-forming member is secured to a proximal end portion of the double-tube member;
FIG. 4C is a partially omitted sectional view of a condition in which an outer needle base for covering the proximal end portion of the double-tube member and the side port-forming member is formed;
FIG. 4D is a partially omitted sectional view of a condition in which a rod-shaped member is inserted in a hollow part of the double-tube member;
FIG. 5A is a partially omitted sectional view of a puncture needle for injecting bone cement according to a first modification;
FIG. 5B is a partially omitted sectional view of a puncture needle for injecting bone cement according to a second modification;
FIG. 6 is a partially omitted perspective view of a puncture needle for injecting bone cement according to a modification;
FIG. 7 is a partially omitted sectional view of a puncture needle for injecting bone cement according to a second embodiment of the present invention;
FIG. 8 is a partially omitted perspective view showing a bulge-forming member and the vicinity thereof in the puncture needle for injecting bone cement according to the second embodiment;
FIGS. 9A to 9D show views for illustrating a method for producing the puncture needle for injecting bone cement according to the second embodiment, in which FIG. 9A is a perspective view showing a hollow cylindrical member having a knurled outer circumferential portion; FIG. 9B is a perspective view of a bulge-forming member; FIG. 9C is a partially omitted sectional view of a double-tube member constituting an outer needle; and FIG. 9D is a partially omitted sectional view of a condition in which a side port-forming member is secured to a proximal end portion of the double-tube member;
FIG. 10A is a perspective view of a first modification of the bulge-forming member;
FIG. 10B is a perspective view of a second modification of the bulge-forming member;
FIG. 10C is a perspective view of a third modification of the bulge-forming member;
FIG. 10D is a perspective view of a fourth modification of the bulge-forming member;
FIG. 10E is a perspective view of a fifth modification of the bulge-forming member;
FIG. 10F is a perspective view of a sixth modification of the bulge-forming member;
FIG. 11 is a partially omitted sectional view of a puncture needle for injecting bone cement according to a third embodiment of the present invention;
FIG. 12 is a partially omitted perspective view showing the vicinity of a proximal end portion of an outer needle in the puncture needle for injecting bone cement according to the third embodiment; and
FIGS. 13A to 13C show views for illustrating a method for producing the puncture needle for injecting bone cement according to the third embodiment, in which FIG. 13A is a partially omitted sectional view showing an outer tube formed with a flange part and a projection; FIG. 13B is a partially omitted sectional view showing a double-tube member constituting the outer needle; and FIG. 13C is a partially omitted sectional view of a condition in which a side port-forming member is secured to a proximal end portion of the double-tube member.

### Description of Embodiments

Preferred embodiments of a puncture needle for injecting bone cement according to the present invention will be described below with reference to the attached drawings. Incidentally, the term "bone cement" as used herein includes not only bone cement (plastic products, etc.) but also bone paste (calcium phosphate products, etc.).

### [First Embodiment]

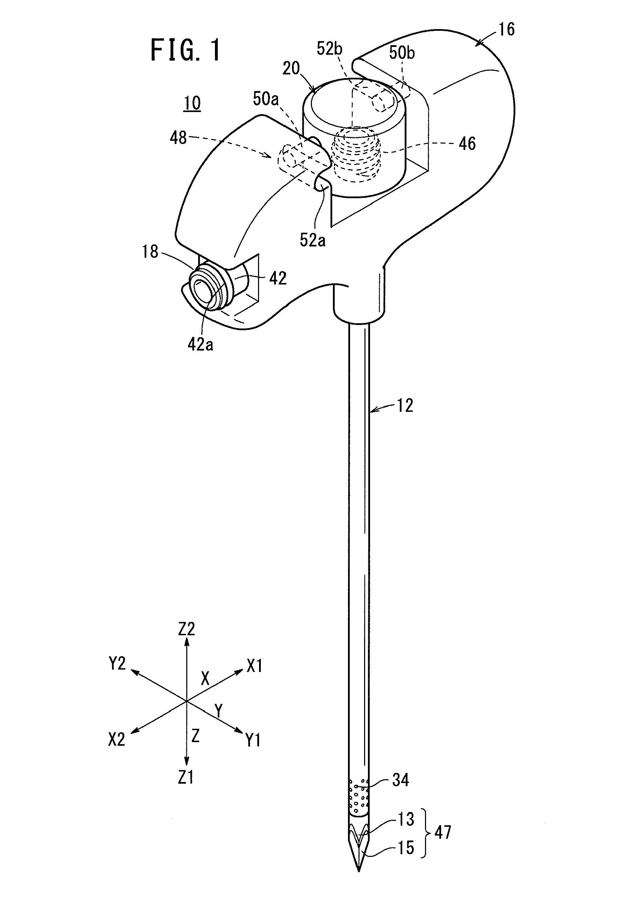

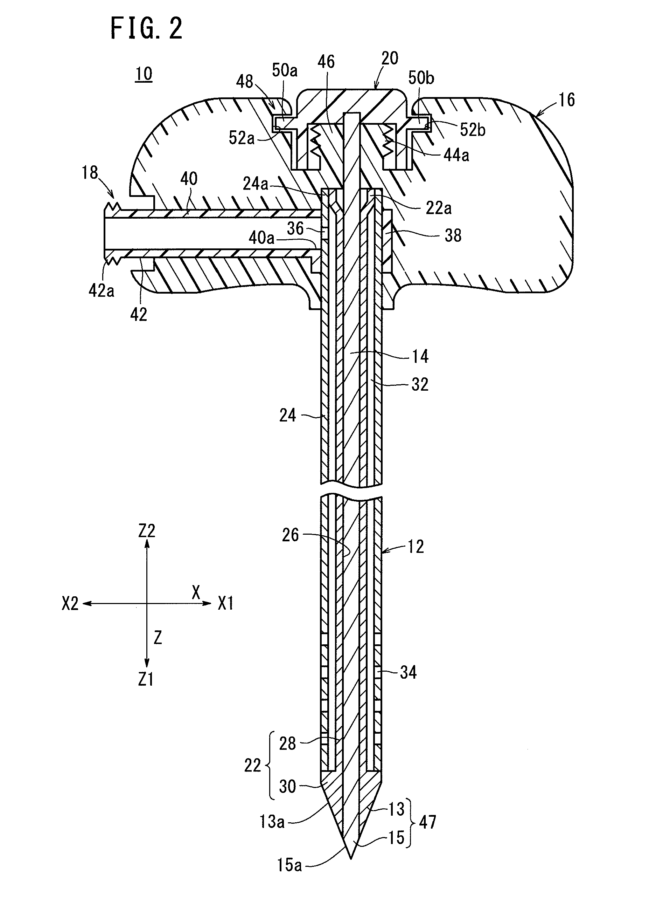

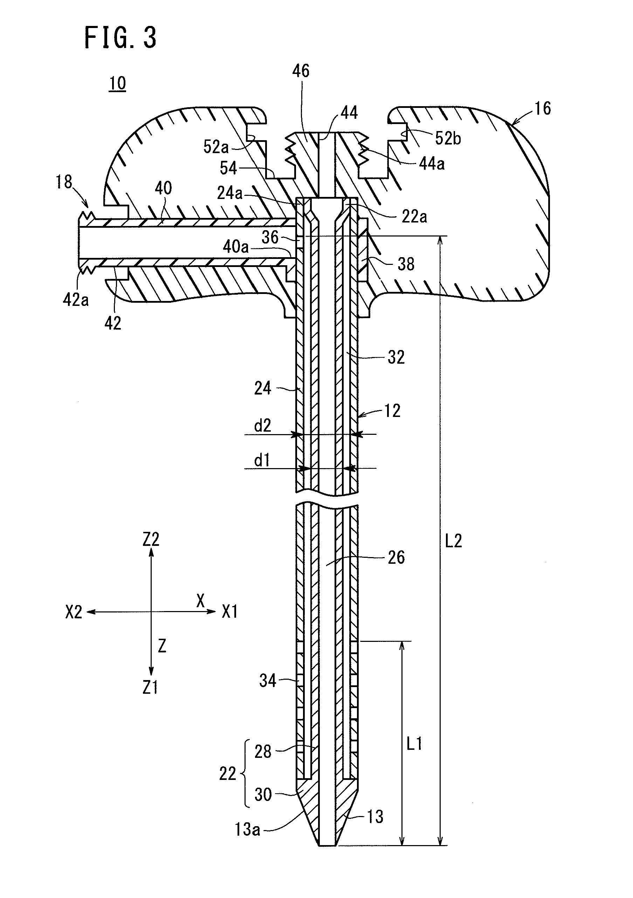

FIG. 1 is a general perspective view of a puncture needle 10 for injecting bone cement (hereinafter also referred to as a "puncture needle 10") according to a first embodiment of the present invention. FIG. 2 is a partially omitted sectional view of the puncture needle 10 shown in FIG. 1. FIG. 3 is a partially omitted sectional view showing a condition in which an inner needle 14 has been pulled out of an outer needle 12.

As shown in FIG. 1, the puncture needle 10 for injecting bone cement includes an outer needle (tubular member) 12 having a hollow structure, an outer needle base 16 secured to a proximal end portion of the outer needle 12, a side port-forming member 18 embedded in the outer needle base 16, an inner needle 14 slidably inserted and passed in the hollow portion of the outer needle 12, and an inner needle base 20 secured to a proximal end portion of the inner needle 14. In FIG. 1, a condition is shown in which the inner needle 14 is inserted into the hollow portion of the outer needle 12.

In the following description, an axial direction of the inner needle 14 and the outer needle 12 will be referred to as a Z-direction, a direction perpendicular to the Z-direction and in which the outer needle base 16 extends will be referred to as an X-direction, and a direction perpendicular to the Z-direction and the X-direction will be referred to as a Y-direction. In FIG. 2, the X-direction is the direction perpendicular to the Z-direction and which is parallel to the surface of the sheet, whereas the Y-direction is the direction perpendicular to the surface of the sheet. In particular, concerning the X-direction, the rightward direction in FIG. 2 will be referred to as an X1 direction, and the leftward direction in FIG. 2 will be referred to as an X2 direction. In particular, concerning the Z-direction, the direction toward the distal end side of the puncture needle 10 will be referred to as a Z1 direction, and the direction toward the proximal end side of the puncture needle 10 will be referred to as a Z2 direction.

The outer needle 12 is a member having a hollow structure and which is open at both ends thereof. The outer needle 12 includes an inner tube 22 through which the inner needle 14 is inserted and passed, and an outer tube 24 that surrounds the inner tube 22. Thus, the outer needle 12 is of a double-tube structure. A sharp cutting edge 13 is formed on the distal end of the outer needle 12.

The material forming the inner tube 22 and the outer tube 24 is not particularly limited, insofar as the material has appropriate strength, so as not to become broken or deformed at times of puncturing a bone or upon withdrawal from a bone. Examples of suitable materials include stainless steel, aluminum alloys, and copper alloys.

As shown in FIG. 3, the inner tube 22 is open at both ends thereof, and is provided therein with a bone cement passage (hollow portion) 26. The bone cement passage 26 functions as a hole through which the inner needle 14 is inserted and passed at a time of assembling the inner needle 14 together with the outer needle 12. The bone cement passage 26 functions as a channel for permitting bone cement to flow therethrough at a time that the bone cement is injected. The length of the inner tube 22 is about 100 to 200 mm. In the exemplary constitution shown in FIG. 3, the inner tube 22 is a hollow cylindrical tube having an inside diameter of about 1.8 to 2.4 mm.

The inner tube 22 is composed of an inner tube section 28 constituting an inner tube wall of the double-tube structure, and a tip 30 provided on a distal end portion of the inner tube section 28. The tip 30 is greater in diameter than the inner tube section 28. A proximal end portion 22a of the inner tube 22 is enlarged in diameter, and is supported by an inner circumferential surface of a proximal end portion 24a of the outer tube 24.

The outer tube 24 opens at both ends thereof, and permits the inner tube 22 to be inserted in the hollow portion thereof. The length of the outer tube 24 is about 100 to 200 mm, and is shorter than the inner tube 22 by the length of the tip 30. The outer tube 24 is a hollow cylindrical tube. An inside diameter d2 of the outer tube 24 is greater than an outside diameter d1 of the inner tube section 28 (except for the proximal end portion 22a thereof). A depressurization passage 32, which extends in the axial direction, is formed between the outer tube 24 and the inner tube section 28. The inside diameter of the outer tube 24, for example, is about 1.9 to 3.2 mm.

A distal end portion of the outer tube 24 is secured to the tip 30 of the inner tube 22, for example, by welding or the like, such that the depressurization passage 32 is closed on the distal end side thereof. In a condition in which the proximal end portion 22a of the inner tube 22 is supported by the inner circumferential part of the proximal end portion 24a of the outer tube 24, the proximal end portion 22a of the inner tube 22 and the proximal end portion 24a of the outer tube 24 overlap with each other in secure contact, whereby the depressurization passage 32 is closed on the rear end side.

The outer tube 24 is provided with a first side hole 34 in the vicinity of a distal end portion thereof. The first side hole 34 is a hole that penetrates through the outer tube 24 from the inside to the outside thereof. Preferably, plural holes are arranged both in the circumferential direction and in the axial direction. The number of the first side holes 34 preferably is 4 to 36, and more preferably, is 10 to 26.

The distance L1 from a distal-most end position of the outer needle 12 to the first side hole 34 located on the most proximal end side (more specifically, to the proximal-most end part of the first side hole 34 in consideration) is set such that, in a condition in which a bone is punctured by the outer needle 12, the first side hole 34 on the most proximal end side is not located outside of the bone, namely, all of the first side holes 34 are located within the bone. More specifically, the distance L1 is equal to or less than 20 mm, and more preferably, is equal to or less than 15 mm.

The first side holes 34 are not necessarily all of the same size, but may be of different sizes. For instance, when a cleaning device is connected to a side port 42 for cleaning the inside of a bone, in order to ensure that the amount of cleaning liquid jetted from the proximal end side of the first side hole 34 in proximity to the side port 42 will not exceed the amount of liquid jetted from the first side hole 34 on the distal end side, the diameters of the side holes located more on the distal end side may be greater. The shapes of the first side holes 34 are not necessarily circular but, for example, may be elliptical or polygonal in shape. In addition, differently shaped first side holes 34 may be provided in combination.

The size of the first side holes 34 is preferably set to permit gas or liquid (for example, an exudate or blood) in the bone to flow smoothly into the outer needle 12. If the first side holes 34 are circular in shape, the diameter thereof preferably is about 0.3 to 0.7 mm. If the first side holes 34 are non-circular in shape, the size at a narrowest portion thereof preferably is about 0.3 to 0.7 mm.

If the first side holes 34 are too small, liquid from inside the bone will easily lead to clogging of the first side holes 34. However, if the lower limit of the size of the first side holes 34 is set as mentioned above, liquid from inside the bone does not easily plug up or clog the first side holes 34. If the size of the first side holes 34 is too large, puncture resistance tends to increase, thereby decreasing the smoothness with which techniques can be performed using the puncture needle. However, if the upper limit of the size of the first side holes 34 is set as mentioned above, an increase in puncture resistance can be restrained.

The outer tube 24 is provided with a second side hole 36 in the vicinity of a proximal end portion thereof. The second side hole 36 is a hole that penetrates through the outer tube 24 from the inside to the outside thereof. The shape of the second side hole 36 is not necessarily circular, but may be, for example, elliptical or polygonal in shape.

The size of the second side hole 36 is preferably set so as to permit gas or liquid (for example, exudate or blood) in the bone to smoothly flow out from the depressurization passage 32. If the second side hole 36 is circular in shape, the diameter thereof preferably is about 1.0 to 2.0 mm. If the second side hole 36 is non-circular in shape, the size at a narrowest portion thereof preferably is about 1.0 to 2.0 mm.

The distance L2 from the distal-most end position of the outer needle 12 to the second side hole 36 (more specifically, to a portion on the most distal end side (Z1-direction side) of the second side hole 36) is set so that, when a bone is punctured by the puncture needle 10, the second side hole 36 is located assuredly outside of the living body. More specifically, the distance L2 is equal to or greater than 80 mm, and more preferably, is equal to or greater than 100 mm.

While only one second side hole 36 is provided in the exemplary constitution shown in FIG. 3, a plurality of second side holes may be provided, as in the modification that will be described later. The first side holes 34 and the second side hole 36 communicate with each other through the depressurization passage 32, which is formed between the inner tube 22 and the outer tube 24. The depressurization passage 32 is a passage provided independently from the bone cement passage 26.

The side port-forming member 18 is formed so as to cover the circumference of the second side hole 36, and to provide communication between the second side hole 36 and the exterior of the outer needle base 16. More specifically, the side port-forming member 18 includes a connecting section 38 in which the outer needle 12 is inserted, and a hollow port body section 40 that extends outwardly (in the X2-direction) from a side portion of the connecting section 38.

The connecting section 38 has a hollow cylindrical shape, the inner diameter of which is approximately equal to the outside diameter of the outer tube 24. An inner circumferential surface of the connecting section 38 and an outer circumferential surface of the outer needle 12 (outer tube 24) are adhered to each other by means of an adhesive. The second side hole 36 confronts a proximal-end-side opening 40a of the port body section 40. The inside diameter of the proximal-end-side opening 40a of the port body section 40 preferably is greater than the second side hole 36. For example, the inside diameter is about 1.5 to 5.0 mm. The inside diameter of the hollow portion of the port body section 40 may be constant along the axial direction, or may become enlarged in diameter in a direction toward the distal end side.

A distal end portion of the side port-forming member 18 is constituted as the side port 42, which protrudes from the outer needle base 16, and to which another device or structure can be attached in a detachable manner. An outer circumferential portion of the side port 42 is formed with a male screw part 42a thereon, which is capable of being screw-engaged with another device or structure.

The material for forming the side port-forming member 18 is not particularly limited. Examples of suitable materials include polyvinyl chloride, polyethylene, polypropylene, cyclic polyolefins, polystyrene, poly(4-methylpentene-1), polycarbonates, acrylic resins, acrylonitrile-butadiene-styrene copolymer, polyesters such as polyethylene terephthalate, polyethylene naphthalate, etc., butadiene-styrene copolymer, and polyamides (for example, nylon 6, nylon 6·6, nylon 6·10, and nylon 12).

The outer needle base 16 is a member that is connected to a proximal end portion of the outer needle 12, and functions as a grip, which can be gripped by a user of the puncture needle 10. The outer needle base 16 is formed by insert molding, so as to cover the proximal end portion of the outer needle 12 as well as the side port-forming member 18. The material for forming the outer needle base 16 is not particularly limited. For example, the material can be selected from among the same materials that were mentioned above as materials for forming the side port 42. The materials forming the outer needle base 16 and the side port 42 are not necessarily the same, but may be different materials.

The outer needle base 16 is formed to protrude (extend) to both sides in a direction (X-direction) perpendicular to the axis of the outer needle 12, and the aforementioned side port 42 that opens at the longitudinally-directed one end portion of the outer needle base 16. A passage 44, which communicates with the bone cement passage 26 in the outer needle 12, is formed in the outer needle base 16. A main port 46 to which the inner needle base 20 is connected is provided at one end portion in the Z2-direction of the outer needle base 16. A hollow portion of the main port 46 constitutes part of the passage 44. A male screw part 44a for detachable engagement with a bone cement injector (for example, a syringe), and which is provided with a lock, is formed on an outer circumferential portion of the main port 46.

In addition, as will be described later, the main port 46 also functions as a receptacle, which is connected to the puncture needle 10 of a syringe for supplying bone cement to the puncture needle 10.

As shown in FIG. 2, the inner needle 14 is constituted from a rod-shaped member, which is inserted in the bone cement passage 26 of the outer needle 12, and which has a sharp cutting edge 15 formed on the distal end thereof. The material for forming the inner needle 14 is not particularly limited, insofar as the material exhibits an appropriate strength so as not to become broken or deformed when a bone is punctured. Examples of suitable materials include stainless steel, aluminum alloys, and copper alloys.

The outside diameter of the inner needle 14 preferably is approximately equal to the inside diameter of the outer needle 12 (the inside diameter of the inner tube 22). More specifically, the outside diameter of the inner needle 14 preferably is set such that the inner needle 14 can be smoothly inserted into the bone cement passage 26, which forms the hollow portion (lumen) of the outer needle 12, and so that substantially no gap is generated between the outer circumference of the inner needle 14 and the inner circumference of the outer needle 12 (the inner circumference of the inner tube 22).

The length of the inner needle 14 is set such that the distal end of the inner needle 14 protrudes slightly from the distal end of the outer needle 12, in a condition in which the inner needle base 20 is connected to the outer needle base 16. In such a condition, in which the inner needle base 20 is connected to the outer needle base 16, a cutting edge surface 15a of the cutting edge 15 of the inner needle 14 is flush with a cutting edge surface 13a of the cutting edge 13 of the outer needle 12. In this manner, the cutting edges 13 and 15 of the outer needle 12 and the inner needle 14 jointly constitute a needle tip 47 of the puncture needle 10.

The inner needle base 20 constitutes a member that is connected to a proximal end portion of the inner needle 14. The outside diameter of the inner needle base 20 is greater than the outside diameter of the inner needle 14. More specifically, the outside diameter of the inner needle base 20 is set so that the inner needle base 20 can easily be pinched with the fingers, and be pushed, pulled and rotated by the user (a health care staff member, such as a doctor or the like). The material that forms the inner needle base 20 is not particularly limited. As suitable materials, materials can be used which are the same as or similar to those for the outer needle base 16. For instance, hard resins such as polycarbonate can be used.

As shown in FIGS. 1 and 2, the puncture needle 10 further includes a lock mechanism 48. The lock mechanism 48 includes a plurality of projection bodies 50a and 50b that project from the outer circumference of the inner needle base 20, and plural engaging grooves 52a and 52b, which are provided in the outer needle base 16 and placed in engagement with the projection bodies 50a and 50b when the inner needle base 20 is connected to the outer needle base 16. The projection bodies 50a and 50b are arranged at symmetrical positions (opposite positions) on the outer circumference of the inner needle base 20.

The engaging grooves 52a and 52b are provided in a side wall that forms a recess 54 of the outer needle base 16, in a state of extending in the thickness direction of the outer needle base 16 (Y-direction). One end on the X2-direction side of the engaging groove 52a is located substantially in the center in the thickness direction of the outer needle base 16, whereas the other end thereof opens on a side surface on one side (Y1 side) of the outer needle base 16. One end of the engaging groove 52b on the X1-direction side is located substantially in the center in the thickness direction of the outer needle base 16, whereas the other end thereof opens on a side surface on the other side (Y2 side) of the outer needle base 16.

The lock mechanism 48 is constituted as described above. Accordingly, when the inner needle base 20 is placed in engagement with the outer needle base 16 for thereby connecting the inner needle base 20 to the outer needle base 16, relative rotation of the inner needle base 20 and the outer needle base 16 causes the projection bodies 50a and 50b to become engaged with the engaging grooves 52a and 52b, respectively. In this instance, the cutting edge surface 15a of the cutting edge 15 of the inner needle 14 and the cutting edge surface 13a of the cutting edge 13 of the outer needle 12 become flush with each other and are maintained in this state.

During injection of bone cement into a bone by use of the puncture needle 10, which is constituted in the foregoing manner, first, a puncture position and a puncture target are determined under imaging-based guidance (i.e., under fluoroscopy or under CT). Thereafter, the puncture needle 10 with the inner needle 14 attached thereto is hit with a hammer, in order to cause the puncture needle 10 to puncture the bone at the puncture target. In this case, the objective bone is a vertebra, for example.

Incidentally, before puncturing a patient using the puncture needle 10, a procedure may be performed in which a tube for supplying a cleaning liquid is connected to the main port 46, whereupon the cleaning liquid is supplied through the passage 44 into the bone cement passage 26 of the inner needle 14 to thereby clean the bone cement passage 26. Similarly, a procedure may be carried out in which a tube for supplying a cleaning liquid is connected to the side port 42, whereupon the cleaning liquid is supplied through the hollow portion of the port body section 40 and the second side hole 36 into the depressurization passage 32 formed between the outer tube 24 and the inner tube section 28.

After the puncture target has been punctured by the puncture needle 10, the inner needle 14 is pulled out from the outer needle 12. In this instance, the first side holes 34 are located within the bone, whereas the second side hole 36 is located outside of the living body.

Next, a syringe filled with bone cement is attached to the main port 46 of the outer needle base 16, whereupon the bone cement in the syringe is injected into the bone through the passage 44 and the bone cement passage 26. In this instance, gas or liquid (for example, exudate or blood) in the bone can enter the depressurization passage 32 through the first side holes 34, so as to be discharged from the living body through the second side hole 36. Thus, the internal pressure inside the bone can be prevented from being raised as a result of injection of the bone cement. Therefore, the bone cement can be prevented from leaking out to the exterior of the bone.

Incidentally, if the side port-forming member 18 is formed from a transparent material (for example, polycarbonate), when liquid that flows out from inside the bone flows into the side port-forming member 18 (i.e., the side port 42), it is possible to visually confirm the interior liquid from outside of the side port-forming member 18. Therefore, flowing out of liquid from inside the bone can be confirmed at an early time, which enables the technique to be performed smoothly. If a suction device is connected to the side port 42, whereby discharging of gas or liquid from inside the depressurization passage 32 is aided, rising of the internal pressure inside the bone can be prevented more effectively.

After the bone cement in the syringe has been injected in a required amount, the syringe is detached. Then, the inner needle 14 is inserted into the passage in the outer needle base 16 and into the bone cement passage 26 of the outer needle 12, whereby bone cement remaining in the passage and the bone cement passage 26 is pushed out into the bone.

In percutaneous vertebral body plasty, in the event that a plurality of puncture needles 10 are used for injecting bone cement, plural puncture needles 10 for injecting bone cement are made to puncture a patient's body, in such a manner that the outer needle bases 16 thereof are arranged mutually in parallel. If the side ports 42 are provided at longitudinally-directed end portions of the outer needle bases 16, as in the puncture needle 10 according to the present embodiment, the side ports 42 of the adjacent puncture needles 10 do not obstruct each other, so that the procedure can be carried out smoothly.

Next, a method for producing the puncture needle 10 constituted in the foregoing manner will be described below. Primarily, a step of forming the outer needle base 16 by insert molding will be described in detail below, whereas other steps will only be described briefly.

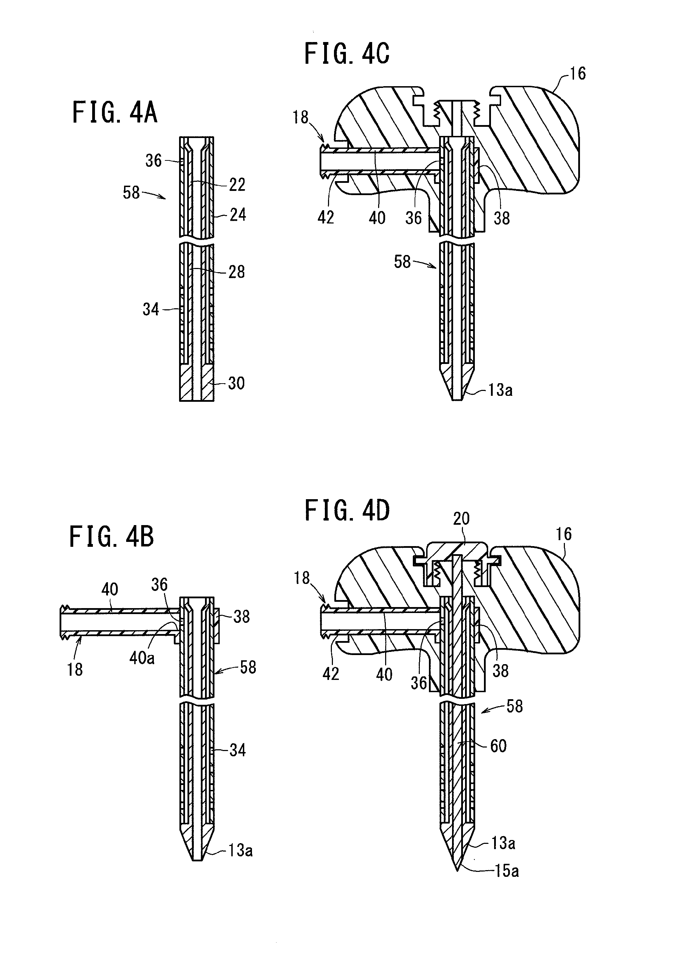

A step of forming the outer needle base 16 at a proximal end portion of the outer needle 12 is carried out by insert molding in the following manner. A double-tube member (tubular member) 58, as shown in FIG. 4A, is a member in a state prior to formation of the cutting edge 13 of the outer needle 12. The double-tube member 58 can be produced, for example, by a method in which the inner tube 22 and the outer tube 24, which are shaped as shown in FIG. 4A, are produced individually, and thereafter, the inner tube 22 is inserted into the outer tube 24, and a distal end portion of the outer tube 24 and the tip 30 of the inner tube 22 are welded to each other. In this case, the inner tube 22 may be obtained by a method in which the tip 30 and the inner tube section 28, which are fabricated separately, are joined to each other by welding or the like.

Next, a securing step is conducted in which the side port-forming member 18 is secured to a proximal end portion of the double-tube member 58, so as to cover the circumference of the second side hole 36. More specifically, as shown in FIG. 4B, initially, a proximal end portion of the double-tube member 58 is inserted into the connecting section 38 of the side port-forming member 18, which is formed by injection molding or the like. At the time of insertion, positional adjustment of the side port-forming member 18 and the double-tube member 58 in the circumferential direction is conducted, so that the second side holes 36 confront the hollow portion of the port body section 40 of the side port-forming member 18. In this case, as mentioned above, the proximal-end-side opening 40a of the port body section 40 is greater than the second side hole 36. This ensures that the second side holes 36 can be made to confront the proximal-end-side opening, even without accurate positional adjustment of the proximal-end-side opening 40a and the second side hole 36 in the circumferential direction. Thus, the position adjusting operation can be simplified.

After the proximal end portion of the double-tube member 58 has been inserted into the connecting section 38 of the side port-forming member 18, an adhesive is applied between the connecting section 38 and the double-tube member 58. Thereafter, the applied adhesive is cured, thereby connecting the side port-forming member 18 and the double-tube member 58 to each other. In this case, it is preferable for a heat-resistant adhesive to be used, so that the adhesive properties of the adhesive are not lost or deteriorated by heat during insert molding, which is carried out at a later time. In this case, the heat resistance temperature of the heat-resistant adhesive must be equal to or greater than the temperature (for example, 200°C) of the molten resin that is injected when insert molding is performed. Examples of suitable heat-resistant adhesives include epoxy adhesives and ceramic adhesives.

A UV-curable adhesive may be used as the adhesive that connects the side port-forming member 18 and the double-tube member 58. More specifically, a process may be adopted in which a UV-curable adhesive is applied between the side port-forming member 18 and the double-tube member 58, whereupon the applied UV-curable adhesive is cured by irradiating the UV-curable adhesive with UV rays. In this case, the side port-forming member 18 may be composed of a transparent member (for example, a polycarbonate having excellent transparency), whereby the UV-curable adhesive can be irradiated with UV rays that are transmitted through the side port-forming member 18. By use of a UV-curable adhesive, it is possible to secure the side port-forming member 18 and the double-tube member 58 to each other in a short time.

Subsequently, as shown in FIG. 4C, an outer needle base forming step is conducted in which the outer needle base 16 for covering the side port-forming member 18 as well as the proximal end portion of the double-tube member 58 is formed by insert molding. In this manner, since the outer needle base 16 is formed by insert molding after the second side holes 36 have been covered by the side port-forming member 18, the outer needle base 16 can be formed by insert molding without closing or deforming the second side holes 36.

Next, as shown in FIG. 4D, a rod-shaped member 60 together with the inner needle base 20 fixed to a proximal end portion thereof is inserted into the hollow portion of the double-tube member 58, and the inner needle base 20 is connected to the outer needle base 16. The inner needle base 20, for example, is formed at the proximal end portion of the rod-shaped member 60 by insert molding. Alternatively, an inner needle base 20, which is preliminarily formed by injection molding or the like, may be secured to an end portion of the rod-shaped member 60 by adhesion, fusion bonding, or the like.

The cutting edge surfaces 13a, 15a may be formed by subjecting the distal ends of the rod-shaped member 60 and the double-tube member 58 to cutting edge surface-forming polishing prior to forming the inner needle base 20 and the outer needle base 16 by insert molding. Alternatively, the rod-shaped member 60 and the double-tube member 58 may be subjected to cutting edge surface-forming polishing simultaneously, after formation of the inner needle base 20 and the outer needle base 16 by insert molding. Further, alternatively, the rod-shaped member 60 and the double-tube member 58 may be subjected to cutting edge surface-forming polishing simultaneously, after the inner needle base 20 is connected to the outer needle base 16, as shown in FIG. 4D. By performing any of the aforementioned cutting edge surface-forming polishing operations, the cutting edge surfaces 13a and 15a are formed, as shown in FIG. 2. Consequently, the outer needle 12 and the inner needle 14 having the cutting edges 13 and 15 on distal ends thereof are formed.

While a preferred embodiment of the present invention has been described above, the invention is not limited to the above embodiment, and it is a matter of course that various alternative arrangements are possible within the scope of the present invention.

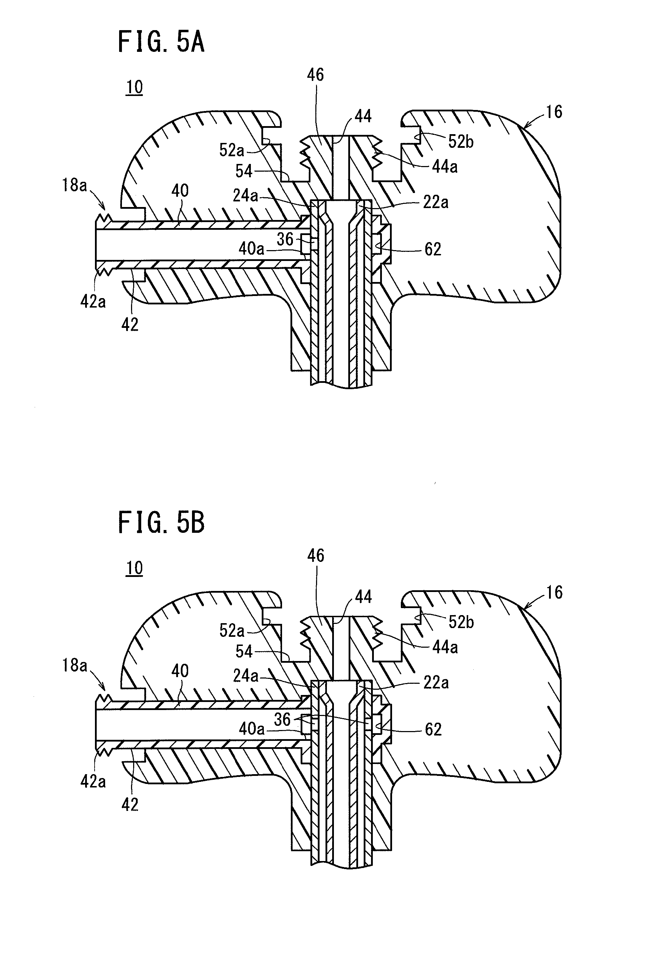

For instance, in a side port-forming member 18a according to the modification shown in FIG. 5A, the connecting section 38 may be provided with a groove 62 on an inner circumferential portion thereof, which extends in the circumferential direction. The second side hole 36 may be formed to confront the groove 62 or the hollow portion of the port body section 40. Such a groove 62 surrounds the outer circumference of a proximal end portion of the outer needle 12, and the groove 62 communicates with the hollow portion of the port body section 40.

If such a groove 62 is provided, even in the event that the side port-forming member 18a is fixed to the outer needle 12 in a condition in which the second side hole 36 is located so as to confront the groove 62, and hence does not confront the hollow portion of the port body section 40, it can be ensured that the second side hole 36 communicates with the hollow portion of the port body section 40 through the groove 62. Therefore, when the proximal end portion of the double-tube member 58 is inserted into the side port-forming member 18a in the production process, it is unnecessary to carry out positional adjustment of the second side hole 36 and the proximal-end-side opening 40a of the port body section 40 in the circumferential direction. Accordingly, the insertion operation can be simplified.

In the case that the side port-forming member 18a formed with the groove 62 therein is adopted, a plurality of second side holes 36 may be provided along the circumferential direction, as shown in FIG. 5B. In the exemplary constitution shown in FIG. 5B, two second side holes 36 are provided on opposite sides (at positions with a phase shift of 180° therebetween). If plural second side holes 36 are arranged along the circumferential direction in this manner, even if one of the second side holes 36 becomes clogged with liquid exuded from inside the bone, it can be ensured that the liquid will still flow out through other second side hole. Accordingly, rising of internal pressure in the bone during injection of bone cement can more reliably be prevented.

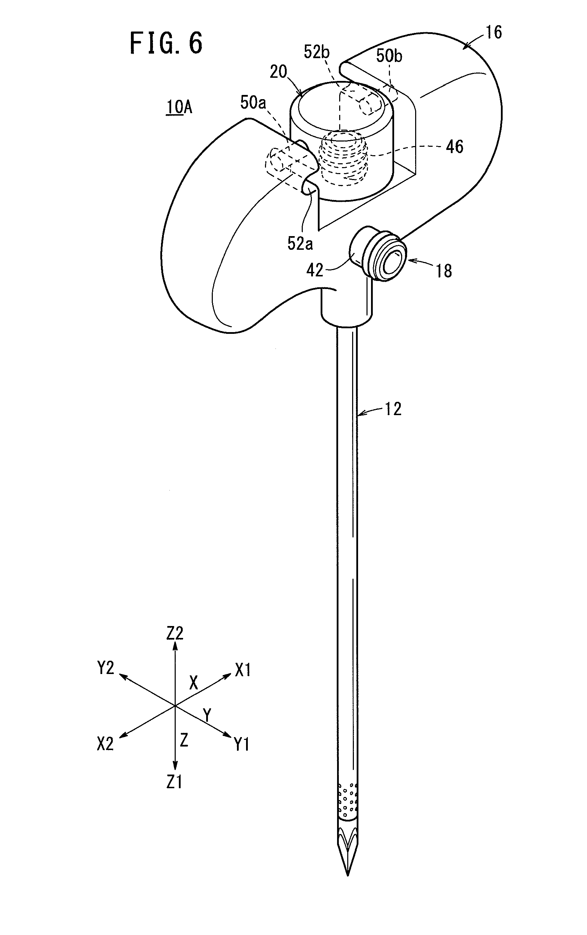

In the puncture needle 10 according to the above-described embodiment, the side port 42 is provided on a longitudinally-directed end portion of the outer needle base 16. However, in a puncture needle 10A for injecting bone cement according to the modification shown in FIG. 6, the side port 42 may be provided on a side surface on one side (a surface on the Y1-direction side) of the outer needle base 16.

In the puncture needle 10 according to the above-described embodiment, the outer needle 12 has a constitution in which the outer tube 24 and the inner tube 22 having the tip 30 at its distal end are welded together. However, the outer needle 12 having such a constitution may be replaced by an outer needle having a constitution in which the outer tube is provided at the distal end thereof with a tapered section, such that the distal end of the inner tube is supported by an inner circumferential portion of the tapered section.

### [Second Embodiment]

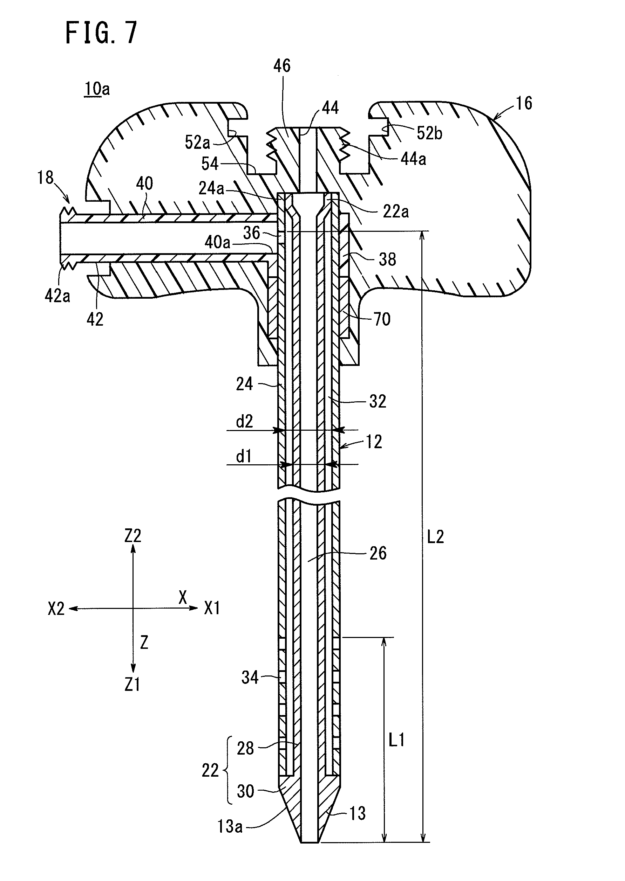

FIG. 7 is a partially omitted sectional view of a puncture needle 10a for injecting bone cement (hereinafter referred to as a "puncture needle 10a") according to a second embodiment. Incidentally, in the puncture needle 10a according to the second embodiment, elements having functions and effects that are the same or similar to those of the puncture needle 10 according to the first embodiment are denoted by the same reference symbols, and detailed descriptions of such features will be omitted.

The puncture needle 10a differs from the puncture needle 10 according to the first embodiment in that a bulge-forming member 70 is secured to an outer circumferential surface of an outer needle 12. The bulge-forming member 70 is a member that bulges from an outer circumferential surface of the outer needle 12 (outer tube 24), and which makes the cross sectional profile orthogonal to the axis of the outer needle 12 non-circular. The bulge-forming member 70 is embedded in an outer needle base 16. As shown in FIG. 7, according to the present embodiment, the bulge-forming member 70 is disposed in the outer needle base 16 at a position more toward the distal end side of the outer needle 12 than the side port-forming member 18.

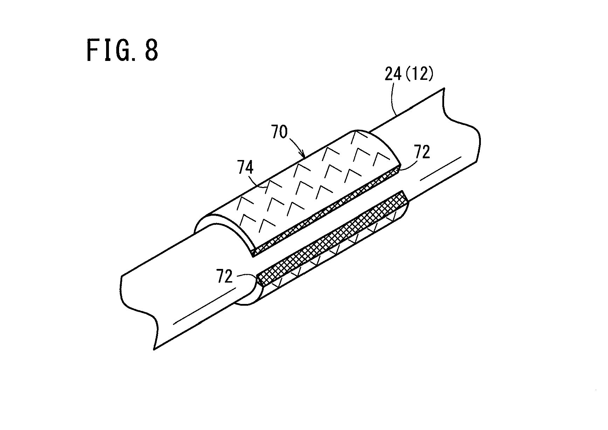

FIG. 8 is a partially omitted perspective view showing the bulge-forming member 70 of the puncture needle 10 and the vicinity around the bulge-forming member 70. The bulge-forming member 70 in the exemplary constitution shown in the figure is C-shaped in cross section and extends along the axial direction of the outer needle 12. The bulge-forming member 70 may be formed of the same material (metal or the like) as that of the outer needle 12, however, the bulge-forming member 70 may also be formed from a material that differs from that of the outer needle 12.

End portions of the C-shaped form of the bulge-forming member 70 and an outer circumferential portion of the outer needle 12 are joined together by joint parts 72, which extend along the axial direction of the outer needle 12. The junction between the bulge-forming member 70 and the outer needle 12 can be provided by any appropriate joining means, such as welding, soldering, adhesion or the like. In the case of the present embodiment, the bulge-forming member 70 is C-shaped in cross section, such that the joint parts 72 can be provided along the axial direction of the outer needle 12. Accordingly, it is possible to secure a large junction area and thus easily obtain a sufficient joint strength.

Knurling (a multiplicity of mountain-shaped projections) 74 is provided on an outer circumferential portion of the bulge-forming member 70. Incidentally, such knurling 74 need not necessarily be provided over the entire outer circumferential portion of the bulge-forming member 70, but may be provided only on a part of the outer circumferential portion.

Meanwhile, it is necessary to prevent the outer needle 12 from slipping out from the outer needle base 16 due to forces that are exerted when the puncture needle 10a, in a state of puncturing a bone, is pulled out from the bone. In addition, it is necessary to prevent the outer needle 12 and the outer needle base 16 from being rotated relative to each other due to a torque, which is exerted when the puncture needle 10a, in a state of puncturing a bone, is rotated. In the case of a puncture needle having a single-tube structure, as in the related art, the outer needle has ample material thickness. Therefore, when a projection or projections (ribs or the like) are formed integrally on an outer circumferential portion of the outer needle by cutting or the like, the projection or projections make it possible comparatively easily to prevent the outer needle from slipping out or from being rotated.

However, in the case of an outer needle 12 having a double-tube structure, in certain cases, the outer tube 24 of the outer needle 12 cannot be provided with sufficient material thickness. Therefore, it is difficult to form a projection or projections on the outer circumferential portion of the outer tube 24 by cutting or the like. In view of this drawback, the puncture needle 10a according to the present embodiment is provided with the bulge-forming member 70, which is secured to an outer circumferential portion of the outer needle 12. According to the puncture needle 10a having such a constitution, the bulge-forming member 70, which is secured to the outer needle 12, is non-circular in cross-section. This ensures that, even if a high torque is exerted between the outer needle 12 and the outer needle base 16, the outer needle 12 and the outer needle base 16 can be prevented from rotating relative to one another. In addition, the bulge-forming member 70 can prevent the outer needle 12 from slipping out in the distal end direction of the outer needle base 16.

Further, in the present embodiment, the bulge-forming member 70 is C-shaped in cross section and extends along the axial direction of the outer needle 12. In addition, end portions of the C-shaped form of the bulge-forming member 70 and the outer circumferential portion of the outer needle 12 are joined together along the axial direction of the outer needle 12. Therefore, since the bulge-forming member 70 constituted in this manner is C-shaped in cross section, the cross sectional profile thereof can easily be made non-circular. In addition, since the joint parts 72 are provided along end portions of the C-shaped form, the bulge-forming member 70 can be secured firmly to the outer needle 12.

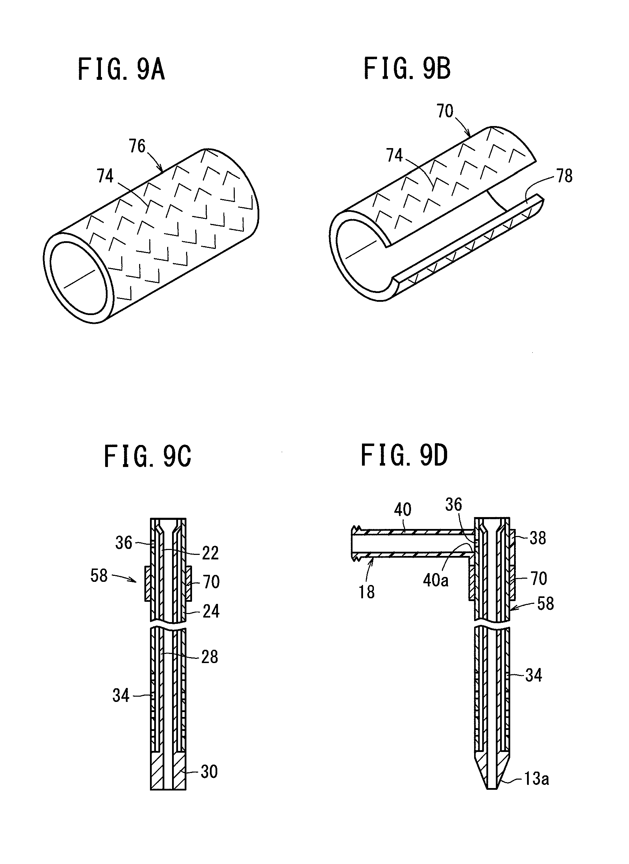

Next, a method for producing the puncture needle 10a according to the present embodiment will be described below. To produce the bulge-forming member 70, initially, a hollow cylindrical member that serves as a base material for the bulge-forming member 70 is prepared. Knurling is formed on an outer circumferential portion of the hollow cylindrical member, whereby the molded article 76 shown in FIG. 9A is produced. Next, a part in the circumferential direction of the molded article 76 is cut out along the axial direction, whereby a gap (cutout) 78, which extends in the axial direction, is formed by the cut-out portion. Consequently, a bulge-forming member 70, which is C-shaped in cross section as shown in FIG. 9B, is produced.

As shown in FIG. 9C, a bulge-adding step is performed in which a double-tube member (tubular member) 58 is inserted into the bulge-forming member 70, whereby the bulge-forming member 70 is secured at a predetermined position of the double-tube member 58. Such securing can be conducted by welding, soldering, or the like, as mentioned above. In this case, the position of the bulge-forming member 70 at which the double-tube member 58 is secured constitutes a position that is located toward the distal end side relative to the second side hole 36 of the double-tube member 58, and a portion of the bulge-forming member 70 is embedded in the outer needle base 16 by insert molding with respect to the outer needle base 16. Incidentally, the double-tube member 58 shown in FIG. 9C can be produced in the same manner as the double-tube member 58 shown in FIG. 4A.

Next, a securing step is conducted in which the side port-forming member 18 is secured to a proximal end portion of the double-tube member 58 so as to cover the circumference of the second side hole 36. More specifically, initially, as shown in FIG. 9D, the double-tube member 58 is inserted, from the proximal end side thereof, into the connecting section 38 of the side port-forming member 18, which is formed by injection molding or the like. During insertion thereof, positional adjustment of the side port-forming member 18 and the double-tube member 58 in the circumferential direction is carried out, so that the second side hole 36 is made to confront the hollow portion of the port body section 40 of the side port-forming member 18.

As shown in FIG. 9D, the side port-forming member 18 preferably is positioned in the axial direction by the bulge-forming member 70. More specifically, the position at which the bulge-forming member 70 is secured to the double-tube member 58 preferably is set such that the second side hole 36 confronts the hollow portion of the port body section 40 of the side port-forming member 18, in a condition in which the connecting section 38 of the side port-forming member 18 is in abutment with the upper end of the bulge-forming member 70. This ensures that positioning of the side port-forming member 18 relative to the double-tube member 58 in the axial direction can easily be carried out speedily and accurately, merely by inserting the double-tube member 58 into the connecting section 38 of the side port-forming member 18, and bringing the connecting section 38 and the bulge-forming member 70 into abutment with each other.

Subsequently, the outer needle base 16 is formed by insert molding so as to cover the side port-forming member 18 and the bulge-forming member 70, as well as the proximal end portion of the double-tube member 58. As a result, the outer needle 12 and the outer needle base 16 are produced, as shown in FIG. 7. The cutting edge surface 13a is formed by subjecting the distal end of the double-tube member 58 to cutting edge surface polishing. In this case, similar to the first embodiment (FIGS. 4A to 4D), formation of the cutting edge surface 13a may be carried out either before or after insert molding of the outer needle base 16.

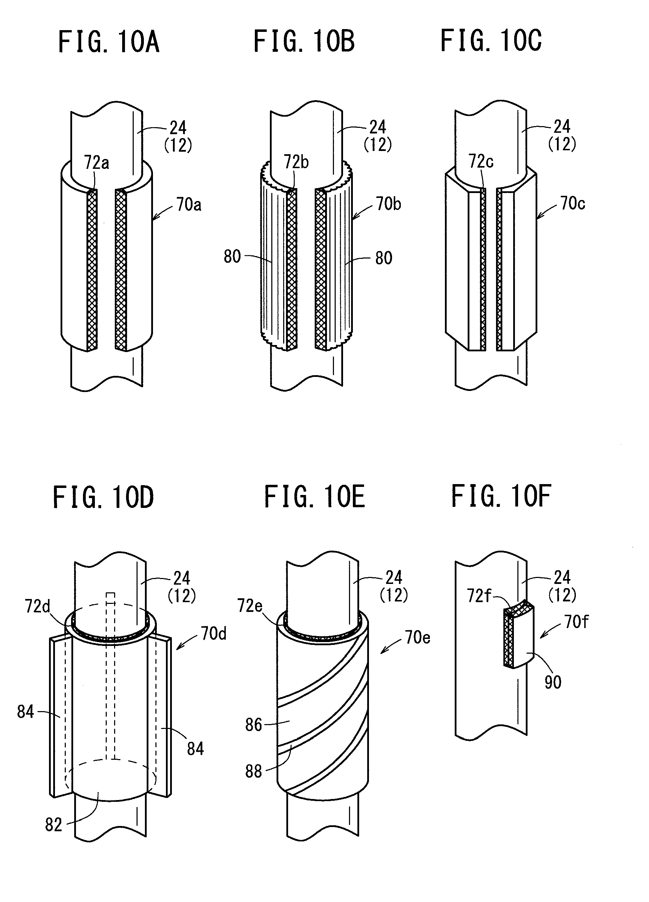

The bulge-forming member 70 shown in FIG. 8 is provided with knurling 74 on the outer circumferential portion thereof. However, another constitution may be adopted, in which the bulge-forming member is C-shaped in cross section, and which includes an outer circumferential portion formed as a smooth cylindrical surface, similar to the bulge-forming member 70a according to the first modification shown in FIG. 10A. In this case, end portions of the C-shaped form and the outer circumferential portion of the outer needle 12 are joined together by joint parts 72a, which extend in the axial direction. According to such a constitution as well, a non-circular cross sectional profile can be realized, thereby preventing the outer needle 12 and the outer needle base 16 from rotating relative to each other.

In this manner, by providing a member which, when provided on the outer circumferential portion of the outer needle 12, makes the cross sectional profile orthogonal to the axis of the outer needle 12 non-circular, relative rotation of the outer needle 12 and the outer needle base 16 can be prevented. Further, based on such a concept, the following second to sixth modifications can also be contemplated.

As shown in FIG. 10B, a bulge-forming member 70b according to a second modification is C-shaped in cross section, and includes ridge-like projections 80, which extend along the axial direction. The ridge-like projections 80 are arranged densely adjacent to one another in the circumferential direction. In this case, end portions of the C-shaped form of the bulge-forming member 70b and the outer circumferential portion of the outer needle 12 are joined together by joint parts 72b that extend in the axial direction.

As shown in FIG. 10C, a bulge-forming member 70c according to a third modification is polygonal in cross-section. In this case, end portions of the C-shaped form of the bulge-forming member 70c and the outer circumferential portion of the outer needle 12 are joined together by joint parts 72c that extend in the axial direction.

According to a fourth modification shown in FIG. 10D, a bulge-forming member 70d includes a cylindrical section 82 and a plurality of ribs 84, which are provided on the outer circumferential surface of the cylindrical section 82 at intervals along the circumferential direction. The plural ribs 84 project outwardly from the outer circumferential surface of the cylindrical section 82 and extend in the axial direction. In this case, both ends of the cylindrical section 82 and the outer circumferential portion of the outer needle 12 are joined together by joint parts 72d that extend in the circumferential direction.

As shown in FIG. 10E, a bulge-forming member 70e according to a fifth modification includes a hollow cylindrical member 86, which is formed with a spiral groove 88 on an outer circumferential portion thereof. In this case, both ends of the hollow cylindrical member 86 and the outer circumferential portion of the outer needle 12 are joined together by joint parts 72e that extend in the circumferential direction. A spiral projection may be provided in place of the spiral groove 88.

As shown in FIG. 10F, a bulge-forming member 70f according to a sixth modification comprises an arcuate member 90, which is formed in the shape of a circular arc of less than 180 degrees. In this case, circumferential edge portions of the bulge-forming member 70f and the outer circumferential portion of the outer needle 12 are joined together by joint parts 72f that extend along circumferential edge portions of the arcuate member 90. The joint parts 72f need not necessarily be provided along the entire circumferential edge portion, but may be provided only partially on the circumferential edge portions of the arcuate member 90.

Incidentally, in the second embodiment, components, which are provided in common with the first and second embodiments, can naturally exhibit operations and effects which are the same or similar as the operations and effects of such common parts in the first embodiment.

In the puncture needle 10a according to the second embodiment, the side port-forming member 18a, as shown in FIGS. 5A and 5B, may be applied in place of the side port-forming member 18. In the puncture needle 10a according to the second embodiment, the side port 42 may be provided on a side surface on one side (a surface on the Y1-direction side) of the outer needle base 16, similar to the puncture needle 10A shown in FIG. 6.

### [Third Embodiment]

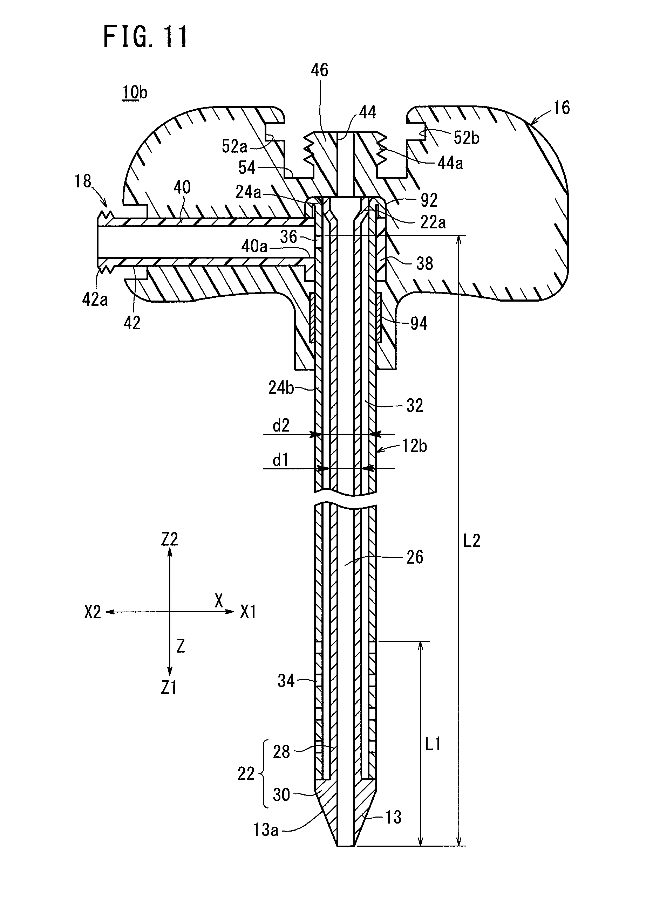

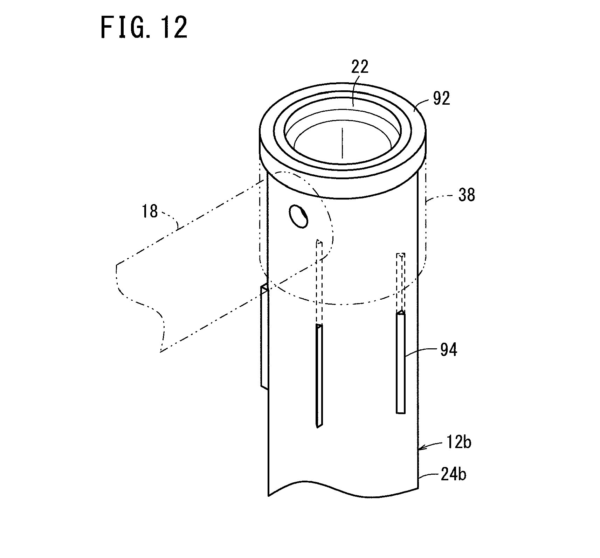

FIG. 11 is a partially omitted sectional view of a puncture needle 10b for injecting bone cement (hereinafter referred to as a "puncture needle 10b") according to a third embodiment. FIG. 12 is a partially omitted perspective view showing the vicinity of a proximal end portion of an outer needle 12b in the puncture needle 10b. Incidentally, in the puncture needle 10b according to the third embodiment, elements that exhibit functions and effects, which are the same or similar as the functions and effects of the elements of the puncture needle 10 according to the first embodiment, are denoted by the same reference symbols, and detailed descriptions of such elements will be omitted.

The puncture needle 10b differs from the puncture needle 10 according to the first embodiment in that the puncture needle 10b has an outer tube 24b (outer needle 12b), which is provided with a flange part 92 and a projection 94, in contrast to the puncture needle 10 having the outer tube 24 (outer needle 12) lacking such a flange part and a projection. As shown in FIGS. 11 and 12, the flange part 92 is formed at a proximal end portion of the outer tube 24b of the outer needle 12b, and the projection 94 is formed in an outer needle base 16 at a position located more toward the distal end side of the outer needle 12 than the side port-forming member 18.

The flange part 92 is a part that bulges radially outward in a ring-like shape at the proximal end portion of the outer tube 24b, which is formed by plastic working. The flange part 92 can be formed, for example, by bending using a press. The outside diameter of the flange part 92 is greater than the inside diameter of a connecting section 38 of the side port-forming member 18. The flange part 92 is placed in abutment with the upper end of the connecting section 38.

The projection 94 is a part that is formed on an outer circumferential portion located more toward the distal end side of the outer tube 24b than the flange part 92, and the projection 94 is embedded in the outer needle base 16. The projection 94 makes the cross sectional profile orthogonal to the axis of the outer tube 24b non-circular, and the projection 94 is formed by plastic working. Formation of the projection 94 can be carried out by coining, for example. Coining ensures that a minute projection 94 (a projection having a height of about 100 µm, for example) can be formed on an outer circumferential portion of the outer tube 24b, even in cases where the material thickness of the outer tube 24b is quite small.

Incidentally, although the size of the projection 94 is exaggerated in FIGS. 11 to 13 to facilitate understanding, the outer diameter of the outer needle 12b at the portion thereof where the projection 94 is provided is approximately equal to or slightly smaller than the inside diameter of the connecting section 38 of the side port-forming member 18.

In the case of the exemplary constitution shown in the figures, a plurality of such projections 94 is formed on the outer circumferential portion of the outer tube 24b at intervals along the circumferential direction. Although it suffices to provide at least one projection 94, it is preferable for a plurality of such projections to be provided, as in the exemplary constitution shown in the figures. The projection 94 is not limited to being one that extends along the axial direction of the outer tube 24b, but a projection may be provided that is inclined relative to the axial direction. For example, a spiral projection may be adopted.

With the puncture needle 10b according to the present embodiment and constituted as described above, the flange part 92 makes it possible to prevent the outer needle 12b from slipping out in the distal end direction of the outer needle base 16. In addition, the projection 94, which makes the cross sectional profile non-circular, makes it possible to prevent relative rotation of the outer needle 12b and the outer needle base 16, even if a high torque is exerted between the outer needle 12 and the outer needle base 16. Further, since the flange part 92 and the projection 94 are formed by subjecting the outer tube 24b to plastic working, the structure for preventing slippage and rotation can easily be provided, without the need for additional component parts.

In the puncture needle 10b according to the present embodiment, the projection 94 is provided more on the distal end side of the outer needle 12b than the side port-forming member 18, such that the projection 94 is provided at a position on the outer needle base 16 which is nearer to the distal end side of the outer needle 12b. Therefore, at this position, separation of the outer needle base 16 and the outer needle 12b from each other is inhibited, and separation can effectively be prevented from spreading toward the proximal end side of the outer needle base 16. Consequently, relative rotation of the outer needle 12b and the outer needle base 16 can more securely be prevented.

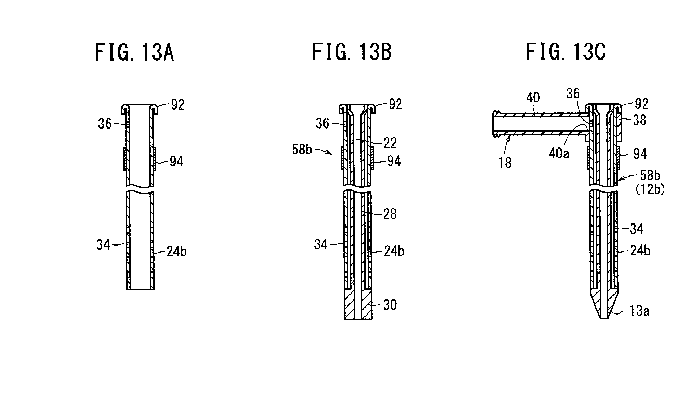

Next, a method for producing the puncture needle 10b according to the present embodiment will be described below. As shown in FIG. 13A, a flange forming step is conducted in which the flange part 92 is formed at a proximal end portion of the outer tube 24b, and a projection forming step is performed in which the outer tube 24b is formed with the projection 94. In this case, the position at which the projection 94 is formed constitutes a position of a portion of the outer tube 24b, which is located toward the distal end side relative to the second side hole 36, and the projection 94 is embedded in the outer needle base 16 by insert molding with respect to the outer needle base 16. The flange forming step and the projection forming step may be performed one after the other in any order, or may be carried out concurrently.

Then, as shown in FIG. 13B, a double-tube member 58b is produced. The double-tube member 58b can be produced, for example, by inserting an inner tube 22 into the outer tube 24b, and then welding a distal end portion of the outer tube 24b and a tip 30 of the inner tube 22 to each other.

Subsequently, a securing step is conducted in which the side port-forming member 18 is secured to a proximal end portion of the double-tube member 58b in order to cover the circumference of the second side hole 36. More specifically, as shown in FIG. 13C, initially, the double-tube member 58b is inserted from the distal end side thereof into the connecting section 38 of the side port-forming member 18, which is formed by injection molding or the like. In this case, since the outside diameter of the outer tube 24b at a portion thereof where the projection 94 is provided is approximately equal to or smaller than the inside diameter of the connecting section 38, the double-tube member 58b can be inserted up to a position at which the connecting section 38 of the side port-forming member 18 moves past the projection 94 and abuts against the flange part 92. Further, upon insertion thereof, positional adjustment in the circumferential direction of the side port-forming member 18 and the double-tube member 58b is conducted, so that the second side hole 36 confronts the hollow portion of the port body section 40 of the side port-forming member 18.

As shown in FIG. 13C, the side port-forming member 18 preferably is positioned in the axial direction by means of the flange part 92. More specifically, preferably, the second side hole 36 confronts the hollow portion of the port body section 40 of the side port-forming member 18, in a condition in which the connecting section 38 of the side port-forming member 18 abuts against the lower end of the flange part 92. This enables positioning of the side port-forming member 18 in the axial direction relative to the double-tube member 58b to be carried out easily, quickly and accurately, merely by inserting the double-tube member 58b into the connecting section of the side port-forming member 18, and then bringing the connecting section 38 and the flange part 92 into abutment with each other.

Next, the outer needle base 16 is formed by insert molding, so as to cover the side port-forming member 18 together with the flange part 92 and the projection 94 of the outer tube 24b, as well as the proximal end portion of the double-tube member 58b. As a result, the outer needle 12 and the outer needle base 16 are produced, as shown in FIG. 11. Incidentally, the cutting edge surface 13a is formed by subjecting the distal end of the double-tube member 58b to cutting edge surface polishing. In this case, similar to the case of the first embodiment (FIGS. 4A to 4D), formation of the cutting edge surface 13a may be performed either before or after insert molding of the outer needle base 16.

Incidentally, in the third embodiment, components thereof, which are in common with components of the first embodiment, can naturally exhibit the same or similar operations and effects as the operations and effects of the common components in the first embodiment.

In the puncture needle 10b according to the third embodiment, the side port-forming member 18a shown in FIGS. 5A and 5B may be applied instead of the side port-forming member 18. Further, in the puncture needle 10b according to the third embodiment, a side port 42 may be provided on a side surface (a surface on the Y1-direction side) on one side of the outer needle base 16, similar to the puncture needle 10A shown in FIG. 6.

## Claims

1. A puncture needle (10, 10A, 10a, 10b) for injecting bone cement into an interior of a bone, comprising:
an outer needle (12, 12b) of a hollow structure and having a bone cement passage (26) therein;
an outer needle base (16) fixed to a proximal end portion of the outer needle (12, 12b) and formed by insert molding;
a side port-forming member (18, 18a) embedded in the outer needle base (16);
an inner needle (14) having a needle tip (15) at a distal end thereof, and which is inserted and passed in a hollow portion of the outer needle (12, 12b) in a slidable manner; and
an inner needle base (20) secured to a proximal end portion of the inner needle (14),
wherein the outer needle (12, 12b) includes a first side hole (34) located on an outer circumferential portion in the vicinity of a distal end portion thereof, a second side hole (36) located on an outer circumferential portion in the vicinity of the proximal end portion thereof, and a depressurization passage (32), which is independent from the bone cement passage (26), and which provides communication between the first side hole (34) and the second side hole (36), and
wherein the side port-forming member (18, 18a) covers a circumference of the second side hole (36) and provides communication between the second side hole (36) and a location outside of the outer needle base (16).

2. The puncture needle (10, 10A, 10a, 10b) for injecting bone cement according to claim 1, wherein:
the side port-forming member (18, 18a) includes a connecting section (38) in which the outer needle (12, 12b) is inserted, and a hollow port body section (40) extending outwardly from a side portion of the connecting section (38);
the second side hole (36) confronts a proximal-end-side opening (40a) of the port body section (40); and
the proximal-end-side opening (40a) of the port body section (40) is greater than the second side hole (36).

3. The puncture needle (10, 10A, 10a, 10b) for injecting bone cement according to claim 1, wherein:
the side port-forming member (18a) includes a connecting section (38) in which the outer needle (12, 12b) is inserted, and a hollow port body section (40) extending outwardly from a side portion of the connecting section (38);
the connecting section (38) is provided on an inner circumferential portion thereof with a groove (62) extending in a circumferential direction; and
the second side hole (36) confronts the port body section (40) or the groove (62).

4. The puncture needle (10a) for injecting bone cement according to claim 1, wherein:
a bulge-forming member (70, 70a to 70f), which bulges from an outer circumferential surface of the outer needle (12) in order to make a cross sectional profile orthogonal to an axis of the outer needle (12) non-circular, is secured to the outer needle (12); and
the bulge-forming member (70, 70a to 70f) is embedded in the outer needle base (16).

5. The puncture needle (10a) for injecting bone cement according to claim 4, wherein:
the bulge-forming member (70, 70a to 70c) comprises a member that is C-shaped in cross section and extends along an axial direction of the outer needle (12); and
end portions of the C-shaped form of the bulge-forming member (70, 70a to 70c) and an outer circumferential portion of the outer needle (12) are joined together along the axial direction of the outer needle (12).

6. The puncture needle (10a) for injecting bone cement according to claim 4, wherein the bulge-forming member (70d, 70e) comprises a tubular member, which is non-circular in cross section.

7. The puncture needle (10a) for injecting bone cement according to claim 4, wherein the bulge-forming member (70, 70a to 70f) is disposed more toward a distal end side of the outer needle (12) than the side port-forming member (18, 18a).

8. The puncture needle (10b) for injecting bone cement according to claim 1, wherein:
a flange part (92) formed by plastic working is provided on a proximal end portion of an outer tube (24b) constituting the outer needle (12b); and
a projection (94), which is formed by plastic working and makes a cross sectional profile orthogonal to an axis of the outer tube (24b) non-circular, is provided on an outer circumferential portion of a part of the outer tube (24b), the part being embedded in the outer needle base (16) and located on a distal end side relative to the flange part (92).

9. The puncture needle (10b) for injecting bone cement according to claim 8, wherein the projection (94) is provided more toward a distal end side of the outer needle (12b) than the side port-forming member (18, 18a).

10. The puncture needle (10, 10A, 10a, 10b) for injecting bone cement according to claim 1, wherein the side port-forming member (18, 18a) is composed of a transparent member.

11. A method for producing a puncture needle (10, 10A, 10a, 10b) for injecting bone cement into an interior of a bone, the method comprising:
a securing step in which a hollow side port-forming member (18, 18a) having a side port (42) at a distal end thereof is secured to a proximal end portion of a tubular member (58, 58b), so as to cover a circumference of a second side hole (36), the tubular member (58, 58b) including a hollow portion that penetrates in an axial direction thereof, a first side hole (34) provided on an outer circumferential portion in the vicinity of a distal end portion thereof, the second side hole (36) provided on an outer circumferential portion in the vicinity of the proximal end portion thereof, and a depressurization passage (32), which is independent from the hollow portion, and which provides communication between the first side hole (34) and the second side hole (36); and
an outer needle base-forming step for forming, by insert molding, an outer needle base (16) that covers the side port-forming member (18, 18a) as well as the proximal end portion of the tubular member (58, 58b).

12. The method for producing a puncture needle (10, 10A, 10a, 10b) for injecting bone cement according to claim 11, wherein, in the securing step, the side port-forming member (18, 18a) and the tubular member (58, 58b) are adhered to each other by a heat-resistant adhesive.

13. The method for producing a puncture needle (10, 10A, 10a, 10b) for injecting bone cement according to claim 11, wherein:
the side port-forming member (18, 18a) is composed of a transparent member; and
the securing step further includes a step of applying an ultraviolet-curable adhesive between the side port-forming member (18, 18a) and the tubular member (58, 58b), and a step of irradiating the applied ultraviolet-curable adhesive with ultraviolet rays in order to cure the ultraviolet-curable adhesive.

14. The method for producing a puncture needle (10a) for injecting bone cement according to claim 11, the method further comprising:
a bulge-adding step in which a bulge-forming member (70, 70a to 70f) for making a cross sectional profile orthogonal to an axis of the tubular member (58) non-circular is secured to an outer tube (24) constituting the tubular member (58),
wherein, in the outer needle base forming step, the outer needle base (16) is formed so as to cover the bulge-forming member (70, 70a to 70f).

15. The method for producing a puncture needle (10b) for injecting bone cement according to claim 11, the method further comprising:
a flange part-forming step in which a flange part (92) is formed by plastic working on a proximal end portion of an outer tube (24b) constituting the tubular member (58b); and
a projection-forming step in which a projection (94) for making a cross sectional profile orthogonal to an axis of the outer tube (24b) non-circular is formed by plastic working on an outer circumferential portion on a distal end side relative to the proximal end portion of the outer tube (24b),
wherein, in the outer needle base-forming step, the outer needle base (16) is formed so as to cover the flange part (92) and the projection (94).
